# EUROPEAN PATENT APPLICATION

(11) **EP 2 422 685 A1**
(43) Date of publication of application: **29.02.2012**
(21) Application number: 11179021.8
(22) Date of filing: 26.08.2011
(51) Int. Cl.: A61B 1/00, A61B 1/015

(54) **Rigid endoscope**

(30) Priority: 30.08.2010 JP 2010192746
(71) Applicant: Fujifilm Corporation, Minato-ku Tokyo 106-8620 (JP)
(72) Inventor: Shibuya, Hiroshi, Kanagawa 258-8538 (JP)
(74) Representative: Klunker . Schmitt-Nilson . Hirsch

(57) **Abstract**

Provided is a rigid endoscope including a fluid pipe line, in which the operability can be improved. A ferrule 50 is provided in a base end part of a hand-side operation part 20 of a rigid endoscope 10, and a leading end part of a fluid pipe line 48 is connected to the ferrule 50. In addition, a cleaning water supply apparatus including a water supply tank 70 and a CO₂ cylinder 76 is connected to a base end part of the fluid pipe line 48, and an observation window 66 and illumination windows 68, 68 are cleaned with cleaning water. Further, the axial direction of the ferrule 50 and the axial direction of a rigid part 28 of an insertion part 22 are set to be parallel to each other, preferably, the same direction, whereby the operability is enhanced. The CO₂ cylinder 76 is connected via a diverter valve 72, and the observation window 66 and the illumination windows 68, 68 are dried with a CO₂ gas. In addition, a base end pipe part 44 to be gripped by an operator and a flange part 46 are provided on the base end side of the hand-side operation part 20, to thereby prevent the fluid pipe line 48 connected to the ferrule 50 from being crushed by the operator by mistake.

## Description

### BACKGROUND OF THE INVENTION

### Field of the Invention

The present invention relates to a rigid endoscope, and more particularly, to a rigid endoscope including a cleaning apparatus which cleans, with cleaning water, an illumination window and an observation window placed on a leading end surface of an insertion part.

### Description of the Related Art

A rigid endoscope which is disclosed in Japanese Patent Application Laid-Open No. 2009-189496 as a rigid endoscope used in a laparoscopic surgery includes a cleaning apparatus which cleans, with cleaning water, a transparent illumination window and a transparent observation window placed on a leading end surface of an insertion part.

The cleaning apparatus includes a water supply sheath into which the insertion part is inserted. A pipe-like connection part is placed in a base end part of the water supply sheath, and a water supply tube is connected to a side part of the connection part. The water supply tube is connected to an external water supply tank, and gas is supplied to the water supply tank via an air supply tube, whereby the cleaning water stored in the water supply tank is supplied to the connection part via the water supply tube by means of a pressure of the gas. The cleaning water supplied to the connection part is introduced into a space between the insertion part and the water supply sheath, and then is guided to the leading end side of the insertion part with this space serving as a water supply channel. As a result, the illumination window and the observation window are cleaned with the cleaning water.

In addition, a universal cable inside of which a light guide, a communication cable, and an electric cable are bundled is connected to an operation part of the rigid endoscope, the operation part being connected to a base end part of the insertion part, and a connector of the universal cable is connected to a light source apparatus and a processor apparatus. The universal cable generally refers to a cable inside of which a light guide, a communication cable, and an electric cable are bundled, but may refer to a cable through which at least one of the light guide, the communication cable, and the electric cable is inserted.

Further, in a rigid endoscope disclosed in Japanese Patent Application Laid-Open No. 05-269079, a water supply port and an air supply port are provided to a connection pipe body of a sheath, and an air supply/water supply pipe line is connected to the connection pipe body via the water supply port and the air supply port. According to this rigid endoscope, when a water supply button provided to the water supply port is opened, a cleaning liquid is supplied to the sheath, and when an air supply button provided to the air supply port is opened, gas is supplied to the sheath.

Japanese Patent Application Laid-Open Nos. 2009-189496 and 05-269079 have the common configuration in that a fluid pipe line such as the water supply tube is connected to the side part of the connection part of the sheath. If the fluid pipe line is connected at this position, there arises a problem that the fluid pipe line becomes obstructive because a hand or an arm of an operator who operates the rigid endoscope hits against the fluid pipe line at the time of operating the rigid endoscope. In particular, the rigid endoscope of Japanese Patent Application Laid-Open No. 2009-189496 includes the universal cable as well as the fluid pipe line, and extending (axial) directions of the fluid pipe line and the universal cable are different from each other, so that the operator is required to operate the rigid endoscope while paying attention to both of the fluid pipe line and the universal cable. Further, when a rotation operation about the axis of the insertion part is performed on the rigid endoscope, the fluid pipe line twines around the sheath, which thus causes a problem in operability.

The present invention has been made in view of the above-mentioned circumstances, and therefore has an object to provide a rigid endoscope including a fluid pipe line, in which the operability can be improved.

### SUMMARY OF THE INVENTION

In order to achieve the above-mentioned object, a rigid endoscope according to the present invention includes: an operation part; an insertion part including a rigid part connected to a leading end part of the operation part; a fluid ejection port which is provided on a leading end surface of the insertion part and ejects a fluid; a pipe line which is provided inside of the insertion part and has a leading end connected to the fluid ejection port; and a ferrule which is provided in a base end part of the operation part and is connected to a base end part of the pipe line, a longitudinal axis of the ferrule being provided so as to be parallel to a longitudinal axis of the rigid part of the insertion part.

According to the present invention, the ferrule is provided in the base end part of the operation part of the endoscope main body, and the leading end part of the fluid pipe line is connected to the ferrule. This makes it possible to solve the problem that a hand or an arm of an operator easily hits against the fluid pipe line at the time of operating the rigid endoscope. In addition, even when a rotation operation about the axis of the insertion part is performed on the rigid endoscope, the fluid pipe line does not twine around the operation part and a sheath, and hence it becomes easier to pull around the fluid pipe line. Accordingly, according to the present invention, the operability of the rigid endoscope can be improved.

Moreover, according to the present invention, the longitudinal axis of the ferrule is provided so as to be parallel to the longitudinal axis of the rigid part of the insertion part, and hence the operability of the rigid endoscope is enhanced.

Further, according to the present invention, it is preferable that the pipe line and the ferrule be placed so that an axis of the pipe line and the longitudinal axis of the ferrule are on substantially the same axis line.

According to the present invention, a cleaning brush can be inserted more easily into the pipe line from the ferrule, and hence cleaning performance of the pipe line using the cleaning brush is enhanced.

It is preferable that the base end part of the fluid pipe line according to the present invention be connected to a cleaning water supply apparatus.

According to the present invention, the cleaning water supply apparatus is connected to the base end part of the fluid pipe line, and hence cleaning water from the cleaning water supply apparatus is supplied to the ferrule. Accordingly, an illumination window and an observation window provided in a leading end part of the insertion part are cleaned with the cleaning water.

It is preferable that the fluid pipe line according to the present invention be connected to a gas supply apparatus via a diverter valve.

According to the present invention, when the diverter valve is operated so that a side of the cleaning water supply apparatus is closed and a side of the gas supply apparatus is opened, gas from the gas supply apparatus is supplied to the ferrule. Accordingly, the illumination window and the observation window provided in the leading end part of the insertion part are dried with the gas. From the point of view of patient protection, it is preferable to use a CO₂ gas as the gas.

It is preferable that the operation part according to the present invention include a flange part on a base end side thereof, and the ferrule be provided in a base end part of the flange part.

According to the present invention, the flange part to be gripped by the operator is provided on the base end side of the operation part, and hence it is possible to prevent the fluid pipe line connected to the ferrule from being crushed by the operator by mistake.

It is preferable that the operation part according to the present invention include: an operation part main body provided with an operation member; and a grip part having: a base end part coupled to the operation part main body via a pipe; and a leading end part connected to the insertion part.

In the rigid endoscope according to the present invention, the operation part may be divided into the operation part main body and the grip part, and the operation part main body and the grip part may be coupled to each other via the pipe. According to this rigid endoscope, the operator grips the operation part main body with one hand, and grips the grip part with another hand. In this state, the operation member in the operation part main body can be operated, and hence the stability of the operation can be achieved.

The ferrule is provided in the base end part of the operation part of the endoscope main body, whereby the pipe line provided inside of the endoscope becomes shorter. Therefore, sterilization performance is enhanced.

According to the rigid endoscope of the present invention, the ferrule is provided in the base end part of the operation part of the endoscope main body, and the leading end part of the fluid pipe line is coupled to the ferrule. Therefore, in the rigid endoscope including the fluid pipe line, the operability can be improved.

### BRIEF DESCRIPTION OF THE DRAWINGS

Fig. 1 is a configuration view illustrating an endoscope system to which a rigid endoscope according to an embodiment is applied;
Fig. 2 is a perspective view illustrating the rigid endoscope illustrated in Fig. 1;
Fig. 3 is a side view illustrating the rigid endoscope illustrated in Fig. 1;
Fig. 4 is a front view illustrating a leading end surface of a leading end hard part of the rigid endoscope illustrated in Fig. 1;
Fig. 5 is a configuration view illustrating a supply system of a physiological saline solution and a CO₂ gas; and
Fig. 6 is a side view illustrating another rigid endoscope in which a hand-side operation part is divided into two.

### DETAILED DESCRIPTION OF THE PREFERRED EMBODIMENTS

Hereinafter, preferred embodiments of a rigid endoscope according to the present invention are described in detail with reference to the attached drawings.

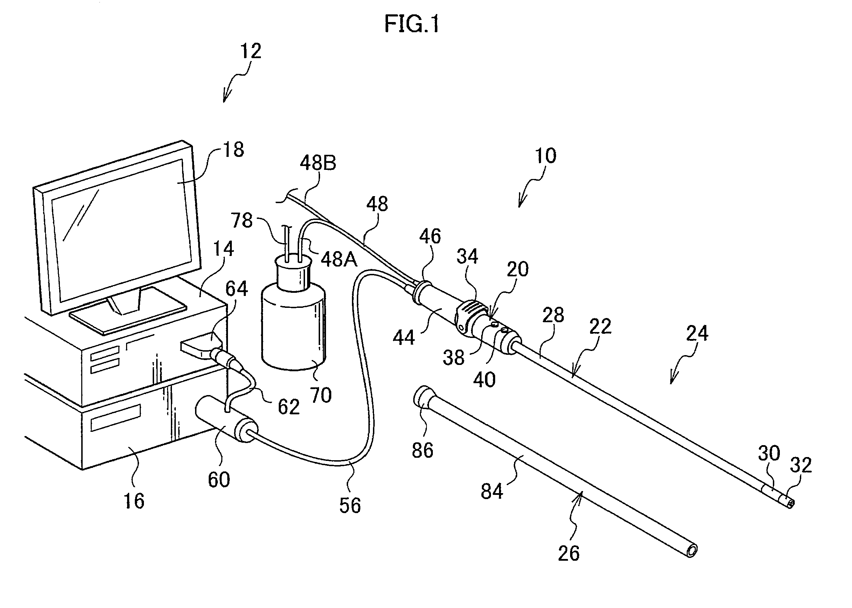

Fig. 1 is a configuration view illustrating an endoscope system 12 to which a rigid endoscope 10 according to an embodiment is applied.

The endoscope system 12 illustrated in Fig. 1 includes the rigid endoscope 10, a processor apparatus 14, a light source apparatus 16, and a monitor 18.

The rigid endoscope 10 includes: an endoscope main body 24 including a hand-side operation part (operation part) 20 to be gripped by an operator and an insertion part 22 to be inserted into an abdominal cavity; and a sheath 26 into which the insertion part 22 is inserted. At the time of an operation, the insertion part 22 is inserted into the sheath 26, and the sheath 26 is inserted into a trocar (not shown) which penetrates through an abdominal wall of a patient. As a result, a leading end of the insertion part 22 is inserted into the abdominal cavity.

The insertion part 22 is connected to a leading end part of the hand-side operation part 20, and includes a rigid part 28, a bending part 30, and a leading end hard part 32 in the stated order from its base end to its leading end. As publicly known, the bending part 30 is configured by coupling a plurality of bending pieces which are each configured into a ring-like shape, and an angle knob (operation member) 34 which is rotatably attached to the hand-side operation part 20 is rotated, to thereby push and pull a wire insertedly provided inside of the insertion part 22 and thus bend the wire in a vertical direction or a horizontal direction. As a result, the leading end hard part 32 can be caused to face a desired direction inside of the abdominal cavity.

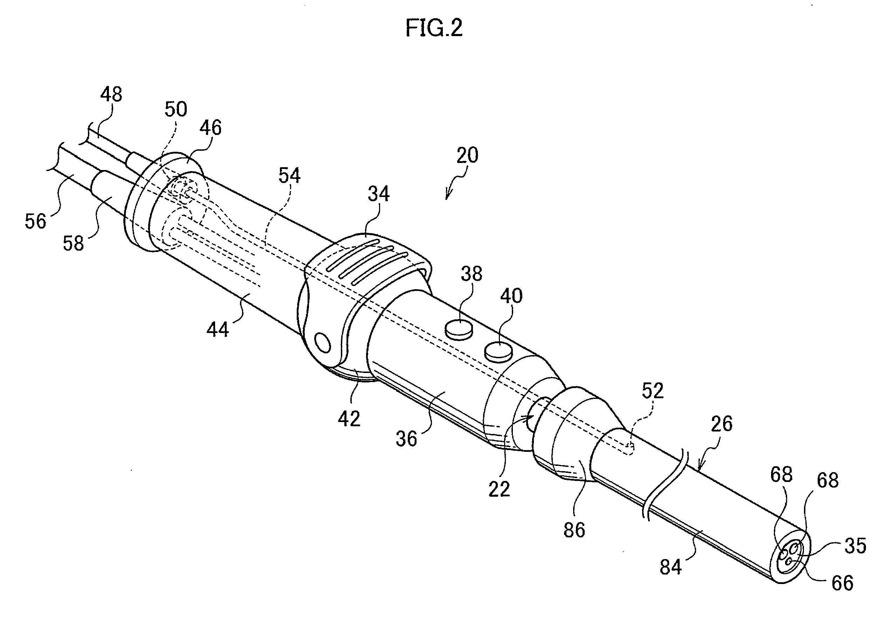

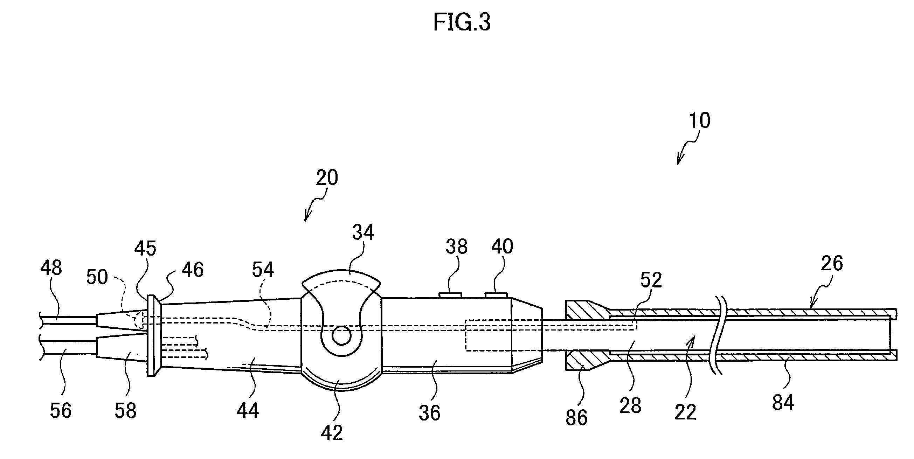

The hand-side operation part 20 is configured into a pipe-like shape having a larger diameter than that of the insertion part 22. As illustrated in Fig. 2 and Fig. 3, the hand-side operation part 20 includes a leading end pipe part 36, a central pipe part 42, and a base end pipe part 44. A water supply button 38 and an air supply button 40 are attached to the leading end pipe part 36, and the angle knob 34 is rotatably attached to the central pipe part 42. In addition, the base end pipe part 44 functions as a grip part to be actually gripped by the operator, and a flange-like grip end 46 is formed at an end of the grip part.

A pipe-like ferrule 50 to which a leading end part of a fluid pipe line 48 is connected is provided on a base end-side end surface 45 of the base end pipe part 44. As illustrated in Fig. 3, the ferrule 50 is connected to an ejection hole 52 which is opened on a circumferential surface of the rigid part 28 of the insertion part 22 via a pipe line 54 indicated by a broken line. The pipe line 54 is provided inside of the hand-side operation part 20, and serves to eject the cleaning liquid and the gas supplied from the fluid pipe line 48 via the ferrule 50, from the ejection hole 52 to the outside. The fluid pipe line 48 will be described later. It should be noted that, in the embodiment, the ejection hole 52 is provided on the circumferential surface of the rigid part 28, but the present invention is not limited thereto. Alternatively, the pipe line 54 may be extended so as to run the entire length of the insertion part 28, and the ejection hole 52 may be provided on a leading end surface 35 of the leading end hard part 32 of the insertion part 28.

Further, a universal cable 56 is connected to the base end-side end surface 45 of the base end pipe part 44 via a bend prevention member 58 adjacently to the ferrule 50. The axial direction of the universal cable 56 and the axial direction (longitudinal axis) of the ferrule 50 are set to be the same direction, and the axial direction of the ferrule 50 and the axial direction (longitudinal axis) of the rigid part 28 of the insertion part 22 are set to be parallel to each other, preferably, the same direction. In addition, the length of the bend prevention member 58 is set to be longer than the length of the ferrule 50 in order to prevent distortion and bend of the fluid pipe line 48.

As illustrated in Fig. 1, an LG connector 60 is connected to an end of the universal cable 56. A signal cable 62 is connected so as to extend from a side part of the LG connector 60, and an electric connector 64 is connected to an end of the signal cable 62. The LG connector 60 is connected to a connector (not shown) of the light source apparatus 16, and the electric connector 64 is connected to a connector (not shown) of the processor apparatus 14. In addition, the processor apparatus 14 is electrically connected to the light source apparatus 16 and the monitor 18, subjects image data taken by the endoscope main body 24 to image processing, and displays the resultant image on the monitor 18.

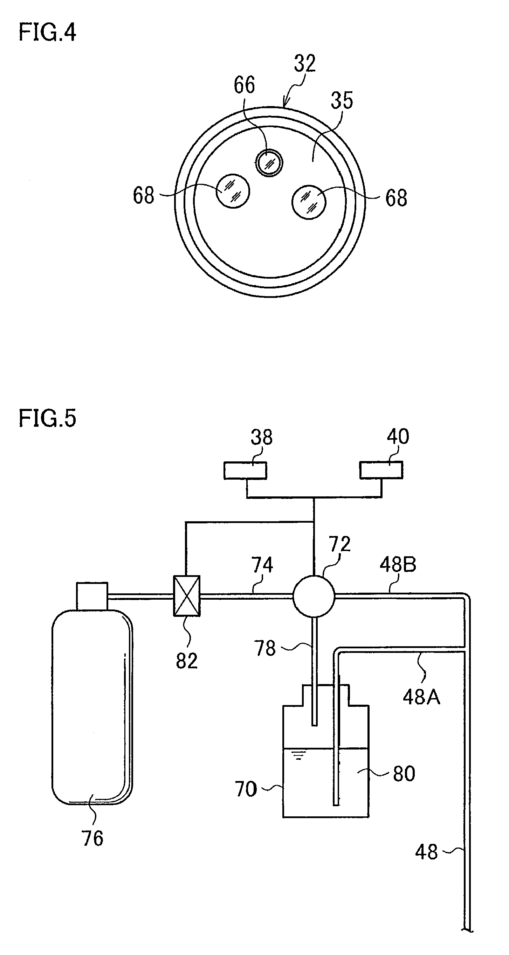

An observation window 66 for taking in subject light is provided on the leading end surface 35 (illustrated in Fig. 4) of the leading end hard part 32 of the insertion part 22. An image pick-up module including an optical system for imaging and a solid state image pick-up element is provided on the inner side of the observation window 66, that is, inside of the leading end hard part 32. A base end part of the signal cable 62 illustrated in Fig. 1 is connected to the image pick-up module. That is, the signal cable 62 is inserted through the universal cable 56, the hand-side operation part 20, and the insertion part 22 to be connected to the image pick-up module.

In addition, as illustrated in Fig. 4, illumination windows 68, 68 for illuminating a subject with illumination light are provided so as to sandwich the observation window 66. A light emitting surface of a light guide is placed on the inner side of the illumination windows 68, 68, that is, inside of the leading end hard part 32 so as to be opposed to the illumination windows 68, 68. The light guide is inserted through the insertion part 22, the hand-side operation part 20, and the universal cable 56 to be connected to the LG connector 60.

Incidentally, as illustrated in Fig. 5, the fluid pipe line 48 connected to the ferrule 50 in a base end part of the hand-side operation part 20 is branched into two. One branched pipe line 48A is attached to a water supply tank 70, and another branched pipe line 48B is connected to a gas pipe 74 via a diverter valve 72. A CO₂ cylinder 76 is connected to the gas pipe 74. In addition, a pipe line 78 for supplying a CO₂ gas of the CO₂ cylinder 76 to the water supply tank 70 is connected to the diverter valve 72. A physiological saline solution 80 which is cleaning water is stored in the water supply tank 70. In addition, a valve 82 for starting/stopping the supply of the CO₂ gas is attached to the gas pipe 74.

Behaviors of the diverter valve 72 and the valve 82 are controlled by operating the water supply button 38 and the air supply button 40.

That is, when the water supply button 38 is turned on, the valve 82 is opened, and the diverter valve 72 is operated, so that the pipe line 78 is opened and the pipe line 48B is closed. As a result, the CO₂ gas from the CO₂ cylinder 76 is supplied to the water supply tank 70 via the gas pipe 74 and the pipe line 78, and hence the physiological saline solution 80 in the water supply tank 70 is supplied to the fluid pipe line 48 via the pipe line 48A. Accordingly, the physiological saline solution 80 passes through the pipe line 54 from the ferrule 50 illustrated in Fig. 2 and Fig. 3 to be ejected from the ejection hole 52 to the outside, then passes through a space between the insertion part 22 and the sheath 26, and flows toward the observation window 66 and the illumination windows 68, 68 illustrated in Fig. 4. As a result, the observation window 66 and the illumination windows 68, 68 are cleaned with the physiological saline solution 80. It should be noted that, when the water supply button 38 is turned off, the valve 82 is closed. Consequently, the cleaning operation with the physiological saline solution 80 is stopped.

In addition, when the air supply button 40 of Fig. 5 is turned on, the valve 82 is opened, and the diverter valve 72 is operated, so that the pipe line 78 is closed and the pipe line 48B is opened. As a result, the CO₂ gas from the CO₂ cylinder 76 is supplied to the fluid pipe line 48 via the gas pipe 74 and the pipe line 48B. Accordingly, the CO₂ gas passes through the pipe line 54 from the ferrule 50 illustrated in Fig. 2 and Fig. 3 to be ejected from the ejection hole 52 to the outside, then passes through the space between the insertion part 22 and the sheath 26, flows toward the leading end hard part 32 of the insertion part 22, and is blown out toward the observation window 66 and the illumination windows 68, 68 illustrated in Fig. 4. As a result, the observation window 66 and the illumination windows 68, 68 are dried with the CO₂ gas. It should be noted that, when the air supply button 40 is turned off, the valve 82 is closed. Consequently, the drying operation with the CO₂ gas is stopped.

As illustrated in Fig. 1, the sheath 26 includes a tube 84 and a connection part 86 connected to a base end part of the tube 84. The insertion part 22 is inserted into the tube 84, and the connection part 86 is detachably connected to the hand-side operation part 20.

Next, a feature of the rigid endoscope 10 according to the embodiment is described.

According to the rigid endoscope 10, the ferrule 50 is provided in the base end part of the hand-side operation part 20, and the leading end part of the fluid pipe line 48 is connected to the ferrule 50. This makes it possible to solve the problem that a hand or an arm of the operator easily hits against the fluid pipe line 48 at the time of operating the rigid endoscope 10.

In addition, even when a rotation operation about the axis of the insertion part 22 is performed on the rigid endoscope 10, the fluid pipe line 48 is less likely to twine around the hand-side operation part 20 and the sheath 26, and hence it becomes easier to pull around the fluid pipe line 48. Accordingly, according to the rigid endoscope 10 of the embodiment, the operability can be improved.

Further, according to the rigid endoscope 10, a cleaning water supply apparatus including the water supply tank 70 and the CO₂ cylinder 76 is connected to the base end part of the fluid pipe line 48, and hence the physiological saline solution 80 from the cleaning water supply apparatus is supplied to the ferrule 50. Accordingly, the observation window 66 and the illumination windows 68, 68 provided in the leading end part of the insertion part 22 can be cleaned with the physiological saline solution 80.

In addition, according to the rigid endoscope 10, the axial direction of the ferrule 50 and the axial direction of the universal cable 56 are set to be the same as the axial direction of the insertion part 22, and hence the operability of the rigid endoscope 10 is further enhanced. Moreover, a cleaning brush can be inserted more easily into the insertion part 22 via the operation part 20 from the ferrule 50, and hence cleaning performance of the ferrule 50 and the pipe line 54 using the cleaning brush is also enhanced.

In addition, in the mode in which: the pipe line 54 is extended so as to run the entire length of the insertion part 28; and the ejection hole 52 is provided on the leading end surface 35 of the leading end hard part 32 of the insertion part 28, the ferrule 50 and the pipe line 54 are placed so that the axial direction of the ferrule 50 and the axial direction of the pipe line 54 are on substantially the same axis line, whereby the cleaning brush can be inserted more easily into the pipe line 54 from the ferrule 50. Accordingly, the cleaning performance of the pipe line 54 using the cleaning brush is also enhanced.

Further, according to the rigid endoscope 10, the CO₂ cylinder (gas supply apparatus) 76 is connected via the diverter valve 72, and hence the observation window 66 and the illumination windows 68, 68 can be dried with the CO₂ gas.

Still further, according to the rigid endoscope 10, the base end pipe part 44 (grip part) to be gripped by the operator is provided on the base end side of the hand-side operation part 20, and hence it is possible to prevent the fluid pipe line 48 connected to the ferrule 50 from being crushed by the operator by mistake.

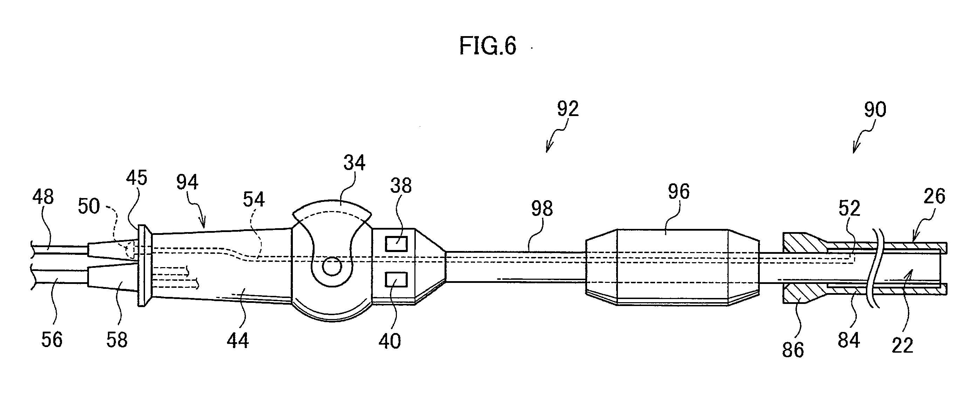

Fig. 6 is a side view illustrating a configuration of a rigid endoscope 90 according to another embodiment, and description is given in the state where identical or similar members to those in the rigid endoscope 10 illustrated in Fig. 1 to Fig. 5 are denoted by the identical reference symbols.

A hand-side operation part 92 of the rigid endoscope 90 illustrated in Fig. 6 includes: an operation part main body 94 provided with the angle knob 34; and a grip part 96, and the operation part main body 94 and the grip part 96 are coupled to each other via a pipe (may be a rigid pipe and a flexible pipe) 98. That is, a base end part of the grip part 96 is coupled to the operation part main body 94 via the pipe 98, and the insertion part 22 is connected to a leading end part of the grip part 96.

According to the rigid endoscope 90, the operator grips the operation part main body 94 with one hand, and grips the grip part 96 with another hand. In this state, the angle knob 34, the water supply button 38, and the air supply button 40 in the operation part main body 94 can be operated, and hence the operation of the rigid endoscope 90 becomes stable.

It should be noted that, in the embodiments, the ejection hole 52 is formed on the circumferential surface of the insertion part 22, but an air supply/water supply pipe may be provided to the sheath, and the air supply/water supply pipe and the pipe line 54 may be connected to each other via a joint. The air supply/water supply pipe is extended up to a leading end part of the sheath 26, an ejection hole is formed at an exit of the air supply/water supply pipe, and the observation window 66 and the illumination windows 68, 68 are cleaned and dried with cleaning water and gas ejected from the ejection hole.

## Claims

1. A rigid endoscope (10) comprising:
an operation part (20);
an insertion part (22) including a rigid part (28) connected to a leading end part of the operation part (20);
a fluid ejection port (52) which is provided on a leading end surface (35) of the insertion part (22) and ejects a fluid;
a pipe line (54) which is provided inside of the insertion part (22) and has a leading end connected to the fluid ejection port; and
a ferrule (50) which is provided in a base end part of the operation part (20) and is connected to a base end part of the pipe line (54), a longitudinal axis of the ferrule (50) being provided so as to be parallel to a longitudinal axis of the rigid part (28) of the insertion part (22).

2. The rigid endoscope (10) according to claim 1, wherein the pipe line (54) and the ferrule (50) are placed so that an axis of the pipe line (54) and the longitudinal axis of the ferrule (50) are on substantially the same axis line.

3. The rigid endoscope (10) according to claim 1 or 2, wherein the base end part of the pipe line (54) is connected to a cleaning water supply apparatus (80).

4. The rigid endoscope (10) according to claim 1, 2, or 3, wherein the pipe line (54) is connected to a gas supply apparatus (76) via a diverter valve (82).

5. The rigid endoscope (10) according to any one of claims 1 to 4, wherein:
the operation part (20) includes a flange part (46) on a base end side thereof; and
the ferrule (50) is provided in a base end part of the flange part (46).

6. The rigid endoscope (10) according to any one of claims 1 to 5, wherein the operation part (20) includes:
an operation part main body provided with an operation member (38, 40); and
a grip part having: a base end part coupled to the operation part main body via a pipe; and a leading end part connected to the insertion part (22).
